Europäisches Patentamt

European Patent Office

Office européen des brevets

(11) Publication number: **0 140 543**
**B1**

# EUROPEAN PATENT SPECIFICATION

(45) Date of publication of patent specification: **27.12.90**

(51) Int. Cl.⁵: **C 12 N 1/38**

(21) Application number: **84306069.0**

(22) Date of filing: **05.09.84**

(54) Method and composition for enhancement of microorganism growth.

(30) Priority: **03.11.83 US 548476**

(43) Date of publication of application:
**08.05.85 Bulletin 85/19**

(45) Publication of the grant of the patent:
**27.12.90 Bulletin 90/52**

(84) Designated Contracting States:
**BE DE FR GB IT NL SE**

(56) References cited:
**CH-A- 494 278**
**FR-A-1 465 355**
**FR-A-2 547 593**
**GB-A-1 027 979**

**CHEMICAL ABSTRACTS, vol. 98, no. 15, April 1983, page 486, abstract no. 124162x, Columbus, Ohio, US; & JP-A-5752834 (Kanegafuci Chemical Industry Co., Ltd) 10-11-1982**

(73) Proprietor: **Becton, Dickinson and Company Mack Centre Drive P.O. Box 2224 Paramus New Jersey 07652-1149 (US)**

(72) Inventor: **Siddioi, Salman H.**
**2126 Eastridge Road**
**Timonium Maryland (US)**
Inventor: **Broman, Rodney L.**
**2127 Buell Drive**
**Fallston Maryland (US)**

(74) Representative: **Ruffles, Graham Keith et al MARKS & CLERK 57-60 Lincoln's Inn Fields London WC2A 3LS (GB)**

Courier Press, Leamington Spa, England.

## Description

The present invention relates generally to a method and composition for enhancing the growth rate and amount of growth of a microorganism in a growth medium. More particularly, the present invention describes a composition and method for the enhancement of the growth rate and amount of growth of mycobacteria in a suitable bacterial growth medium.

The cultivation for propagating microorganisms by providing the proper nutritional and environmental conditions is well known. In general, the contributing factors for suitable propagating conditions include the nature and level of nutrients provided in the growth media, pH, temperature, and aeration. Other factors which must be controlled include the salt concentration and osmotic pressue of the medium and in some cases, such special factors as light for photosynthetic organisms.

A suitable growth medium must contain all the nutrients required by the microorganism which is to be cultivated. In general, the following nutrients must be provided:

(1) hydrogen donors and acceptors;
(2) a carbon source;
(3) a nitrogen source;
(4) minerals, such as sulphur and phosphorous and trace elements such as potassium, magnesium, calcium and iron.

In addition, it is known that certain growth factors are required. A growth factor is an organic compound which a microorganism must contain in order to grow but which it is unable to synthesize. Many microorganisms, when provided with the nutrients listed above, are able to synthesize all of the organic constituents of their protoplasm, including amino acids, vitamins, purines and pyrimadines, fatty acids and other compounds. Each of these essential compounds is synthesized by a discrete sequence of enzymatic reactions and each enzyme is produced under the control of a specific gene. When an organism undergoes a gene mutation resulting in failure of one of these enzymes to function, the chain is broken and the end product is no longer produced. The microorganism must then obtain that compound from the environment and the compound has become a growth factor for the organism.

Different microorganisms vary widely in their nutritional and growth factor requirements. In CH—494278 there is described the fermentation of various microorganisms using a hydrocarbon as a carbon source and in the presence of surface active agents. GB—A—1027979 discloses the termination of BCG in the presence of glycerine as carbon source and a particular class of nonionic surface active agents. The present invention is directed to the provision of a substance which enhances the growth rate and amount of growth of certain microorganisms in a growth medium containing nutrients required for their growth.

It has been determined that the addition of polyoxyethylene stearate to a growth medium containing nutrients, including bovine serum albumin for mycobacterium microorganisms enhances the growth rate and amount of growth of the microorganisms in a liquid medium. In addition to the increase in growth, as observed visually or on stained smear, this enhancement can be observed by a higher $CO_2$ production which is significant for detection of the growth of the microorganism in automated radioimmunoassay systems.

According to the invention there is provided a method for the enhancement of growth of a mycobacterium microorganism, the method comprising adding the microorganism to a culture medium containing bovine serum albumin and an effective amount of polyoxyethylene stearate and subjecting the culture medium to conditions suitable for growth of the microorganism.

The single figure of the accompanying drawings is a graph of a growth medium used to grow a strain of M. tuberculosis. The growth medium is designated BACTEC 12A. The lower line of graph shows the use of the 12A medium by itself. The upper line of the graph shows the use of the 12A medium in combination with polyoxyethylene stearate.

Mycobacteria in general, and M. tuberculosis and M. bovis in particular, are very slow growing organisms. It takes about two to three weeks to grow most of these organisms on the culture media conventionally used. There have been several efforts to find a medium or a substance which can enhance the growth and reduce the time factor. A report on the stimulating effect of RNA on the growth of mycobacteria has resulted in the Gruft modification of Lowenstein Jensen medium (Gruft, H. 1971, Isolation of Acid-Fast Bacilli From Contaminated Specimens, Health Lab Science 8:79). The enhancement of growth by RNA, however, is still questionable and there has been no demonstration of any significant effect through the use of such RNA addition to the growth medium.

A method for detecting bacteria using the detection of radioactive $^{14}C$ is described in United States Patent No. 3,935,073 to Waters. A growth medium to be used along with the apparatus described in the Waters patent and which is suitable for the culture of mycobacteria contains the following nutrients at the indicated level:

| 7H9 broth base (containing potassium and sodium phosphates, sodium glutamate, sodium citrate, ammonium sulphate, pyridoxine, ferric ammonium citrate, magnesium sulphate, zinc sulphate, copper sulphate, biotin and calcium chloride obtained from BBL Microbiology Systems in Cockeysville, Maryland) | 0.47% |
|---|---|
| Bovine serume albumin | 0.5% |
| Caseine Hydrolysis | 0.1% |
| Catalase | 96 units/vial |
| $^{14}$C labelled substrate | 2 Ci/vial |
| Deionized water | 2 qds. |
| Final pH | 6.8 ± 0.1 |

The addition of polyoxyethylene stearate to the above identified growth medium enhances growth of mycobacteria to a significant degree. In general, polyoxyethylene stearate is added to the growth medium in concentration of from 10 micrograms to 10,000 micrograms per milliliter of medium. The enhancement is directly proportional to the concentration of polyoxyethylene stearate. In particular, it is found that about 50 to about 500 micrograms per milliliter is a preferred range at which the growth and production of $^{14}CO_2$ is enhanced without having any negative effect on the acid fastness or other physiological properties of the organisms.

The polyoxyethylene stearate solubilizes in an aqueous growth medium. Consequently the physical form of the polyoxyethylene stearate is not critical. The polyoxyethylene stearate can be added in the form of pellets, granules or powder or can be sprayed onto the interior surface of a culture vial. The polyoxyethylene stearate can be used in both broth and agar growth media. If used in agar the polyoxyethylene stearate should be solubilized prior to adding the agar.

As an example of the growth enhancement properties of the method and composition of the present invention, a growth medium was prepared having the following components at the indicated level:

| 7H9 broth base | 0.47% |
|---|---|
| Bovine serume albumin | 0.5% |
| Caseine Hydrolysis | 0.1% |
| Catalase | 96 units/vial |
| $^{14}$C labelled substrate | 2 Ci/vial |
| Deonized water | 2 qds. |
| Final pH | 6.8 ± 0.1 |

The growth medium described above was divided into two portions. In one-half, polyoxyethylene stearate (granular form) was added at a level of one hundred micrograms per milliliter. The other half of the growth medium was used without any further additions.

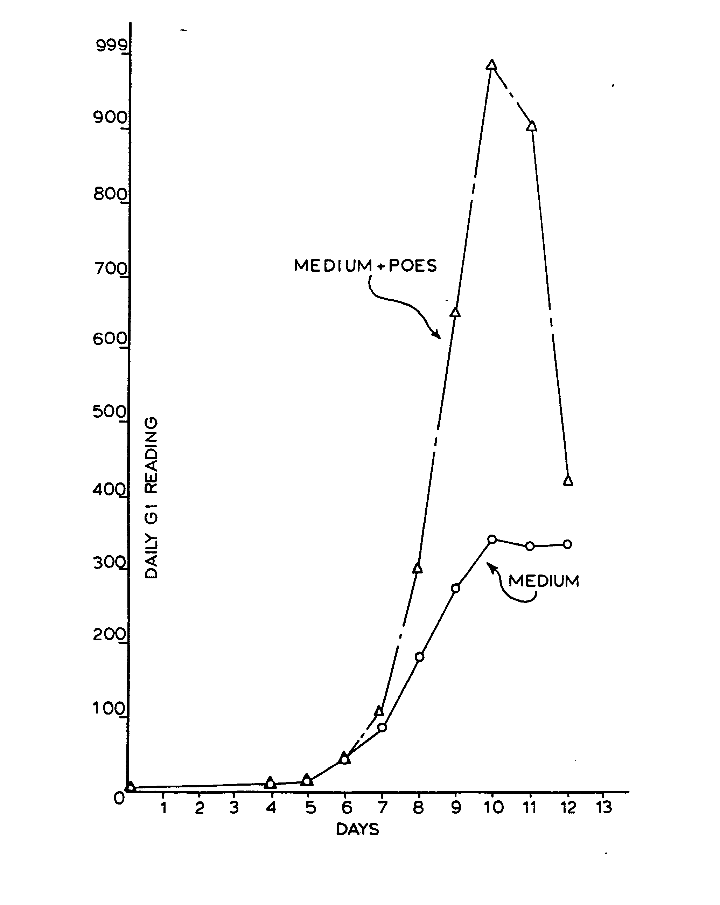

A suspension of *M. tuberculosis*, strain H37Rv, equivalent to MacFarland No. 1 standard, was diluted 1:500 and 0.1 ml was inoculated into each bottle containing 2 ml of growth medium. Eight bottles each of the growth medium alone and the growth medium plus the polyoxyethylene stearate were inoculated. All of the bottles were read on an apparatus similar to that described in the Waters patent previously mentioned. The readings were taken daily for twelve days. The daily growth index (GI) values recorded by the apparatus indicated the $^{14}CO_2$ production from the $^{14}$C-labelled substrate present in the medium. The results of the readings were used to prepare the two plots of Figure 1.

The growth of *M. tuberculosis* was also observed visually as well as by making smears and staining by the Ziehl Neelsen method for acid-fast bacteria.

It is evident from the plots of Figure 1 that the growth curve of *M. tuberculosis* in the growth medium to which polyoxyethylene stearate has been added is significantly enhanced from that of the growth medium without such addition. The enhancement effect on the growth of *M. tuberculosis* in the medium is clearly shown once the growth index reaches around 100—150 on day 6 through day 8. At this point, the increase in mycobacterial growth becomes very abrupt. At an equivalent growth index reading, the amount of growth, as observed visually, is also significantly higher in the polyoxyethylene containing medium than the regular 12A medium. When smears are prepared from the growth medium containing polyoxyethylene stearate and stained by the Ziehl Neelsen method, acid-fast bacteria are more dispersed in long ropey serpentine cords and there are more in number.

Data of primary isolation (recovery) of mycobacteria from clinical specimens (sputum) is included in Table 1. The enhancement of growth and GI output of a positive mycobacterial culture in the presence of polyoxyethylene stearate is evident from these two examples. After the day 7 GI output, which also indicates the rate and amount of growth, is significantly higher in the 12A medium with polyoxyethylene stearate as compared to 12A medium without it.

Similar enhancement effect has been noticed with *M. bovis* and many mycobacteria other than *M. tuberculosis* (MOTT or atypical mycobacteria).

## TABLE 1
### Stimulation of Growth and $^{14}CO_2$ Production in 12A Medium with 100 µg/ml POES
### (Clinical Isolate of *M. tuberculosis*)

|  | GI Readings Day | | | | | | | | | |
|---|---|---|---|---|---|---|---|---|---|---|
| Culture | 1 | 2 | 3 | 4 | 5 | 6 | 7 | 8 | 9 | 10 |
| #136 12A | 2 | 1 | 2 | 4 | 10 | 26 | 50 | 104 | 184 | 298 |
| #136 12A + POES | 1 | 1 | 0 | 3 | 10 | 18 | 51 | 154 | 429 | 999 |
| #129 12A | 1 | 1 | 0 | 10 | 20 | 57 | 119 | 227 | 365 | 533 |
| #129 12A + POES | 1 | 0 | 2 | 3 | 11 | 34 | 97 | 279 | 751 | 999 |

## Claims

1. A method for the enhancement of growth of a mycobacterium microorganism, the method comprising adding the microorganism to a culture medium containing bovine serum albumin and an effective amount of polyoxyethylene stearate and subjecting the culture medium to conditions suitable for growth of the microorganism.

2. A method in accordance with claim 1 wherein the culture medium is a broth type medium.

3. A method in accordance with claim 1 wherein the culture medium is an agar type medium.

4. A method in accordance with claims 1, 2 or 3 wherein the polyoxyethylene stearate is added to the culture medium at a level of from 10 to 10,000 micrograms per millilitre medium.

5. A method in accordance with claim 4 wherein the polyoxyethylene stearate is added to said culture medium at a level of from 50 to 500 micrograms per millilitre.

6. A growth medium having enhanced growth properties for mycobacterium microorganisms, the medium comprising a culture medium containing bovine serum albumin and an effective amount of polyoxyethylene stearate.

7. A growth medium in accordance with claim 6 which is a broth type.

8. A growth medium in accordance with claim 6 which is an agar type.

9. A growth medium in accordance with claims 6, 7 or 8 wherein the polyoxyethylene stearate is present at a level of from 10 to 10,000 micrograms per millilitre of medium.

10. A growth medium in accordance with claim 9 wherein the polyoxyethylene is present at a level of from 50 to 500 micrograms per millilitre.

## Patentansprüche

1. Verfahren zur Steigerung des Wachstums eines Mycobakterium-Mikroorganismus, wobei das Verfahren umfaßt: Zugabe des Mikroorganismus zu einem Kulturmedium, welches Rinderserumalbumin und eine wirksame Menge Polyoxyethylenstearat enthält und Unterwerfen des Kulturmediums unter Bedingungen, die für das Wachstum des Mikroorganismus geeignet sind.

2. Verfahren nach Anspruch 1, wobei das Kulturmedium ein Medium vom Brühetap ist.

3. Verfahren nach Anspruch 1, wobei das Kulturmedium ein Medium vom Agartyp ist.

4. Verfahren nach einem der Ansprüche 1, 2 oder 3, wobei das Polyoxyethlenstearat dem Kulturmedium mit einem Anteil von 10 bis 10 000 Mikrogramm pro Milliliter Medium zugesetzt wird.

5. Verfahren nach Anspruch 4, wobei das Polyoxyethlenstearat dem Kulturmedium mit einem Anteil von 50 bis 500 Mikrogramm pro Milliliter zugesetzt wird.

6. Wachstumsmedium mit gesteigerten Wachstumseigenschaften für Mycobakterium-Mikroorganismen, wobei das Medium ein Kulturmedium umfaßt, das Rinderserumalbumin und eine wirksame Menge von Polyoxyethlenstearat enthält.

7. Wachstumsmedium nach Anspruch 6, das vom Brühetyp ist.

8. Wachstumsmedium nach Anspruch 6, das vom Agartyp ist.

9. Wachstumsmedium nach den Ansprüchen 6, 7 oder 8, wobei das Polyoxyethylenstearat mit einem Anteil von 10 bis 10 000 Mikrogramm pro Milliliter Medium vorhanden ist.

10. Wachstumsmedium nach Anspruch 9, bei dem das Polyoxyethylen mit einem Anteil von 60 bis 500 Mikrogramm pro Milliliter vorhanden ist.

**Revendications**

1. Procédé pour augmenter la croissance d'un microorganisme mycobactérien, le procédé consistant à ajouter le microorganisme à un milieu de culture contenant de l'albumine de sérum bovin et une quantité effective de stéarate de polyoxyéthylène et à soumettre le milieu de culture à des conditions adaptées à la croissance du microorganisme.

2. Procédé selon la revendication 1 dans lequel le milieu de culture est un milieu du type bouillon.

3. Procédé selon la revendication 1 dans lequel le milieu de culture est un milieu du type agar-agar.

4. Procédé selon la revendication 1, 2 ou 3 dans lequel le stéarate de polyoxyéthylène est ajouté au milieu de culture à un taux de 10 à 10 000 microgrammes par millilitre de milieu.

5. Procédé selon la revendication 4 dans lequel le stéarate de polyoxyéthylène est ajouté audit milieu de culture à un taux de 60 à 500 microgrammes par millilitre.

6. Milieu de culturer possédant des propriétés de croissances accrues pour les microorganismes mycobactériens, le milieu comprenant un milieu de culture contenant de l'albumine de sérum bovin et une quantité effective de stéarate de polyoxyéthylène.

7. Milieu de culture selon la revendication 6 qui et du type bouillon.

8. Milieu de culture selon la revendication 6 qui et du type agar-agar.

9. Milieu de culture selon la revendication 6, 7, ou 8 dans lequel le stéarate de polyoxyéthylène est présent à un taux de 10 à 10 000 microgrammes par millilitre de milieu.

10. Milieu de culture selon la revendication 9 dans lequel le polyoxyéthylène est présent à un taux de 50 à 500 microgrammes par millilitre.

EP 0 140 543 B1